# EUROPÄISCHE PATENTSCHRIFT

(11) **EP 1 972 358 B1**
(45) Veröffentlichungstag und Bekanntmachung des Hinweises auf die Patenterteilung: **19.09.2012**
(21) Anmeldenummer: 07005695.7
(22) Anmeldetag: 20.03.2007
(51) Int. Cl.: A61M 35/00

(54) **Vorrichtung zum Auftragen fliessfähiger Stoffe**
Device for applying flowable substances
Dispositif destiné à l'application de matières pouvant s'écouler

(43) Veröffentlichungstag der Anmeldung: 24.09.2008
(73) Patentinhaber: Sulzer Mixpac AG, 9469 Haag (CH)
(72) Erfinder: Sògaro, Alberto C., 61476 Kronberg (DE)
(74) Vertreter: advotec.

(56) Entgegenhaltungen:
- WO-A2-02/48002
- DE-U1- 20 020 049
- US-A- 4 747 719
- US-A- 5 112 152
- US-A- 5 704 906
- US-A1- 2004 134 815

## Beschreibung

Die Erfindung betrifft eine Vorrichtung zum Auftragen flüssiger, pastenartiger und anderer fließfähiger Stoffe.

Eine derartige Vorrichtung ist aus der Praxis bekannt und beispielsweise in Form eines Pinsels ausgebildet, mittels dessen der fließfähige Stoff, der beispielsweise eine Flüssigkeit wie Azeton oder ein anderer im Medizin- oder Dentalbereich eingesetzter Stoff ist, appliziert werden kann. Der fließfähige Stoff bzw. die Flüssigkeit wird in der Regel in einem Behälter vorgehalten, in den der Pinsel bei der Anwendung eingetaucht wird, so dass dessen Pinselhaar mit der Flüssigkeit beaufschlagt wird. Der Pinsel und der Behälter werden hierbei von einem Anwender getrennt vorgehalten.

Das Dokument US 5 112 152 offenbart eine Vorrichtung zum Auftragen fließfähiger Stoffe nach dem Oberbegriff des Anspruchs 1.

Der Erfindung liegt die Aufgabe zugrunde, eine Vorrichtung zum Auftragen flüssiger, pastenartiger und anderer fließfähiger Stoffe zu schaffen, die sich durch eine besonders einfache Anwendbarkeit auszeichnet.

Diese Erfindung ist erfindungsgemäß durch die Vorrichtung mit den Merkmalen des Anspruchs 1 gelöst.

Erfindungsgemäß wird also eine Vorrichtung zum Auftragen eines flüssigen, pastenartigen oder anderen fließfähigen Stoffes vorgeschlagen, umfassend eine Verpackung mit einem einen Nassbereich aufweisenden Aufnahmeabschnitt zur Aufnahme des Stoffes und einem einen Trockenbereich begrenzenden Verschlussabschnitt, der über eine Reißlinie von dem Aufnahmeabschnitt getrennt ist, wobei der Nassbereich von dem Trockenbereich mittels eines Trennelements getrennt ist, das eine durch ein Verschlusselement verschlossene Öffnung aufweist, das nach einem Aufreißen der Verpackung entlang der Reißlinie von dem Trennelement lösbar ist.

Gemäß einer bevorzugten Ausführungsform wird eine Vorrichtung zum Auftragen eines flüssigen, pastenartigen oder anderen fließfähigen Stoffes vorgeschlagen, umfassend eine Verpackung mit einem einen Nassbereich aufweisenden Aufnahmeabschnitt zur Aufnahme des Stoffes und einem einen Trockenbereich begrenzenden Verschlussabschnitt, der über eine Reißlinie von dem Aufnahmeabschnitt getrennt ist, einen Applikator, der von der Verpackung vollständig aufgenommen ist und mit einem Applikationsabschnitt und einem Griffabschnitt versehen ist, der in dem Trockenbereich der Verpackung liegt, wobei der Nassbereich von dem Trockenbereich mittels eines Trennelements getrennt ist, das eine durch den Applikator verschlossene Öffnung zum Eingriff des Applikationsabschnitts des Applikators in den Nassbereich aufweist.

Die Vorrichtung nach der Erfindung weist also in Form des Aufnahmeabschnitts einen Nassbereich und in Form des Verschlussabschnitts einen Trockenbereich auf, in welchem der Griffabschnitt des beispielsweise als Pinsel oder dergleichen ausgebildeten Applikators angeordnet ist.

Der Applikationsabschnitt, der unter anderen ein Pinselhaar, eine Beflockung oder ein Schaumstoff sein kann und an den Griffabschnitt angeklebt oder angespritzt sein kann, taucht bei einer speziellen Ausführungsform in den Nassabschnitt ein und ist somit schon vor der Anwendung der Vorrichtung mit dem zu applizierenden Stoff beaufschlagt. Bei der Anwendung wird zunächst der Verschlussabschnitt von dem Aufnahmeabschnitt durch Aufreißen des Behälters entlang der Reißlinie abgetrennt. Dann muss der Applikator nur aus der Dichteinrichtung und dem Aufnahmeraum für den zu applizierenden Stoff gezogen werden. Der Stoff kann dann direkt und unmittelbar auf eine zu beaufschlagende Fläche aufgetragen werden.

Der zu applizierende Stoff ist innerhalb der Vorrichtung in dem Aufnahmeraum gefangen und kann sich nicht in der gesamten Verpackung, insbesondere nicht in dem Verschlussabschnitt bzw. Trockenbereich der Verpackung verteilen. Nach dem Auftrennen der Verpackung sieht ein Anwender den sauberen Griffabschnitt des Applikators.

Um das Trennelement sicher innerhalb der Verpackung zu positionieren, ist diese bei einer bevorzugten Ausführungsform der Vorrichtung nach der Erfindung mit der Verpackung versiegelt. Dies ist insbesondere möglich, wenn das Trennelement aus Kunststoff gefertigt ist und die Verpackung ebenfalls aus einem einen Kunststoff aufweisenden Werkstoff gefertigt ist.

Bei der erfindungsgemäße Ausführungsform, die sich beispielsweise zur Aufnahme von Azeton oder dergleichen eignet, ist das Trennelement ein einen Aufnahmeraum begrenzender Behälter, der eine Primärverpackung für den fließfähigen Stoff bildet, und dass die Verpackung eine Sekundärverpackung für den fließfähigen Stoff bildet.

Beispielsweise ist der Behälter ein einseitig geschlossenes Rohrstück, das den Aufnahmeraum begrenzt und das im Bereich seines geschlossenen Endes mit der Verpackung verbunden ist. Insbesondere ist das Rohrstück im Bereich seines geschlossenen Endes mit der Verpackung verschweißt. Das Rohrstück bildet dann die Primärverpackung für den fließfähigen Stoff, wobei die Verpackung eine Sekundär- bzw. Umverpackung darstellt.

Bei einer speziellen Ausführungsform taucht der Applikationsabschnitt des Applikators in den Nassbereich ein und durchgreift der Griffabschnitt des Applikators die Öffnung des Trennelements dichtend.

Alternativ kann der Applikationsabschnitt des Applikators auch in dem Trockenbereich liegen und der Griffabschnitt des Applikators mit seinem dem Applikationsabschnitt abgewandten Ende die Öffnung des Trennelements verschließen. Diese Ausführungsform bietet sich insbesondere an, wenn der von der Vorrichtung aufgenommene fließfähige Stoff den Applikationsbereich des Applikators angreifen kann und beispielsweise eine Verklebung zwischen dem Applikationsbereich und dem Griffbereich bei längerer Einwirkung auflösen würde.

Der Griffabschnitt kann einen Verschlussstopfen für die Öffnung des Trennelements bilden. Alternativ kann der Griffabschnitt einstückig mit dem Trennelement hergestellt sein und im Bereich der Öffnung über eine Sollbruchstelle von dem Trennelement getrennt sein, wobei ein Abtrennen des Applikators von dem Trennelement dessen Öffnung freigibt.

Die Vorrichtung nach der Erfindung lässt sich besonders einfach herstellen, wenn die Verpackung aus zwei in ihren Randbereichen umlaufend miteinander versiegelten Folienzuschnitten gebildet ist. Die Folienzuschnitte können insbesondere jeweils aus einer Verbundfolie, beispielsweise aus einem Aluminiumlaminat gebildet sein. Eine aus einem Aluminiumlaminat gefertigte Verpackung ist auch zur sicheren Aufnahme von Azeton oder anderen aggressiven Stoffen geeignet.

Zur Sicherung der Dichteinrichtung in der Verpackung können die Folienzuschnitte in Höhe der Dichteinrichtung eine quer zur Achse des Applikators verlaufende Siegelnaht aufweisen.

Die Reißlinie, die keine eindeutig definierte Linie darstellen muss sondern sich je nach ausgeübter Aufreißkraft ändern kann, ist bei einer zweckmäßigen Ausführungsform der Vorrichtung nach der Erfindung durch mindestens eine seitliche Aufreißkerbe der Folienzuschnitte festgelegt. Die Aufreißkerbe liegt zweckmäßigerweise im Bereich der umlaufenden Siegelnaht der beiden Folienzuschnitte.

Das Trennelement kann bei einer speziellen Ausführungsform, bei der keine Primär- und Sekundärverpackung erforderlich ist und der Aufnahmeraum für den fließfähigen Stoff direkt von der Verpackung bzw. deren Folienzuschnitten begrenzt ist, auch ein platten- bzw. scheibenartiger Kunststoffeinsatz sein, der eine mittige Bohrung bzw. Ausnehmung aufweist, deren Durchmesser mit einem Schaft der Applikationseinrichtung korrespondiert. Der Kunststoffeinsatz kann einen linsenförmigen Grundriss aufweisen. Bei einer speziellen Ausführungsform der Vorrichtung nach der Erfindung ist die Dichteinrichtung ein Rohrstück, das von der Applikationseinrichtung durchgriffen ist. Dieses in Form einer Hülse ausgebildete Rohrstück ist mit dem Material der Verpackung versiegelt.

Um den Applikator gesichert in der Dichteinrichtung zu halten, kann die Applikationseinrichtung eine Innennut aufweisen, in die die Dichteinrichtung eingreift. Alternativ kann natürlich auch die Applikationseinrichtung einen Ringwulst aufweisen, der in eine Ringnut der Dichteinrichtung eingreift.

Ferner kann der Griffabschnitt des Applikators eine Riffelung aufweisen, die den Sitz des Applikators in dem Trennelement sichert.

Weitere Vorteile und vorteilhafte Ausgestaltungen der Vorrichtung nach der Erfindung sind der Beschreibung, der Zeichnung und den Ansprüchen entnehmbar.

Drei Ausführungsbeispiele einer Vorrichtung nach der Erfindung sind in der Zeichnung schematisch vereinfacht dargestellt und werden in der nachfolgenden Beschreibung näher erläutert. Es zeigt
- Fig. 1: eine schematische Darstellung einer beutelartig ausgebildeten Vorrichtung nach der Erfindung mit einer Applikationseinrichtung;
- Fig. 2: eine schematische Darstellung einer zweiten Ausführungsform einer beutelartig ausgebildeten Vorrichtung nach der Erfindung; und
- Fig. 3: eine schematische Darstellung einer Ausführungsform einer ebenfalls beutelartig ausgebildeten Vorrichtung nach der Erfindung;
- Fig. 4: eine schematische Darstellung einer Ausführungsform einer ebenfalls beutelartig ausgebildeten Vorrichtung nach der Erfindung; und
- Fig. 5: eine schematische Darstellung einer Ausführungsform einer ebenfalls beutelartig ausgebildeten Vorrichtung nach der Erfindung.

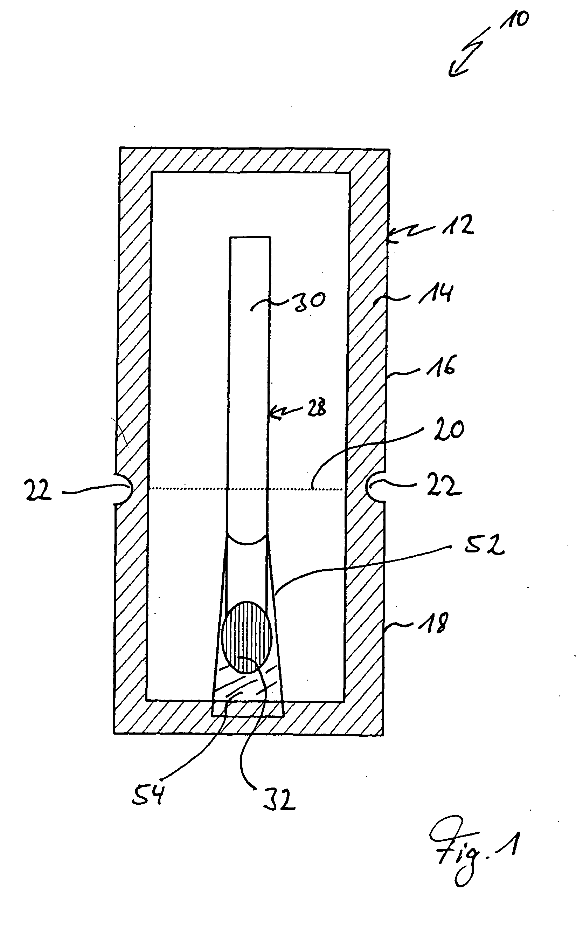

In Fig. 1 ist eine Vorrichtung 10 zur Aufnahme und Applikation einer flüssigen Substanz, wie Azeton dargestellt, die beispielsweise im Dentalbereich eingesetzt werden kann.

Die Vorrichtung 10 umfasst eine Verpackung 12 mit im Wesentlichen rechteckigem Grundriss, die aus zwei in einem umlaufenden Randbereich 14 miteinander versiegelten Aluminiumverbundfolien hergestellt ist. Die Verpackung 12 umfasst einen Verschlussabschnitt 16, der einen Trockenbereich begrenzt, und einen einen Nassbereich aufweisenden Aufnahmeabschnitt 18, die über eine Reißlinie 20 voneinander getrennt sind, welche durch Kerben 22 festgelegt ist, die beidseits im Bereich des eine Randsiegelnaht darstellenden Randbereichs 14 angeordnet sind. Die Reißlinie 20 kann durch eine Materialschwächung der Folienzuschnitte gebildet sein oder sich auch erst beim Aufreißen der Verpackung von einer der Kerben 22 aus ergeben.

Die Vorrichtung 10 umfasst des Weiteren ein einen Behälter darstellendes Röhrchen 52, das einen den Nassbereich bildenden Aufnahmeraum 54 des Aufnahmeabschnitts 18 der Verpackung 12 begrenzt und ein Trennelement zwischen dem Trockenbereich und dem Nassbereich bildet.

Der Applikator 28 ist mit einem Applikationsabschnitt 32 in das Röhrchen 52 eingesteckt, so dass der Applikationsabschnitt 32 in den von dem Röhrchen aufgenommenen fließfähigen Stoff eintaucht.

Das Röhrchen 52, das also den Nassbereich der Vorrichtung 50 begrenzt und einen Behälter bildet, ist mit seinem dem Griffabschnitt 30 der Applikationseinrichtung 28 abgewandten Ende, d. h. mit seinem Fußende, in die umlaufende Siegelnaht der beutelartigen Verpackung 12 eingeschweißt bzw. platt geschweißt. Dadurch, dass zumindest der dem Fußende abgewandte Endbereich des Röhrchens 52 einen Innendurchmesser aufweist, der mit dem Durchmesser des Griffabschnitts 30 des Applikators 28 korrespondiert, dichtet das Röhrchen 52 selbst den den Nassbereich bildenden Aufnahmeraum 54 gegenüber dem innerhalb der Verpackung 12 liegenden und den Griffabschnitt 30 des pinselartigen Applikators und das Röhrchen 52 umgebenden Trockenbereich ab. Damit kann keine Flüssigkeit aus dem Aufnahmeraum 54 in den Trockenbereich austreten und sich innerhalb der Verpackung 12 verteilen. Nach dem Aufreißen der Verpackung 12 entlang der Reißlinie 12, die durch die Kerben 22 definiert ist, liegt somit ein sauberer Griffabschnitt 30 des Applikators 28 vor.

Bei der Anwendung der Vorrichtung 10 reißt ein Benutzer die Verpackung 12 entlang der Reißlinie 20 auf und nimmt den den Trockenbereich 16 begrenzenden Verschlussabschnitt ab, so dass der saubere Griffabschnitt 30 des Pinsels 28 freiliegt. Dann zieht der Benutzer den Pinsel 28 aus dem Röhrchen 52 heraus, so dass der mit dem zu applizierenden Stoff beaufschlagte Applikationsabschnitt 32 des Pinsels 28 zum Auftragen des Stoffes in Kontakt mit einer Applikationsfläche gebracht werden kann.

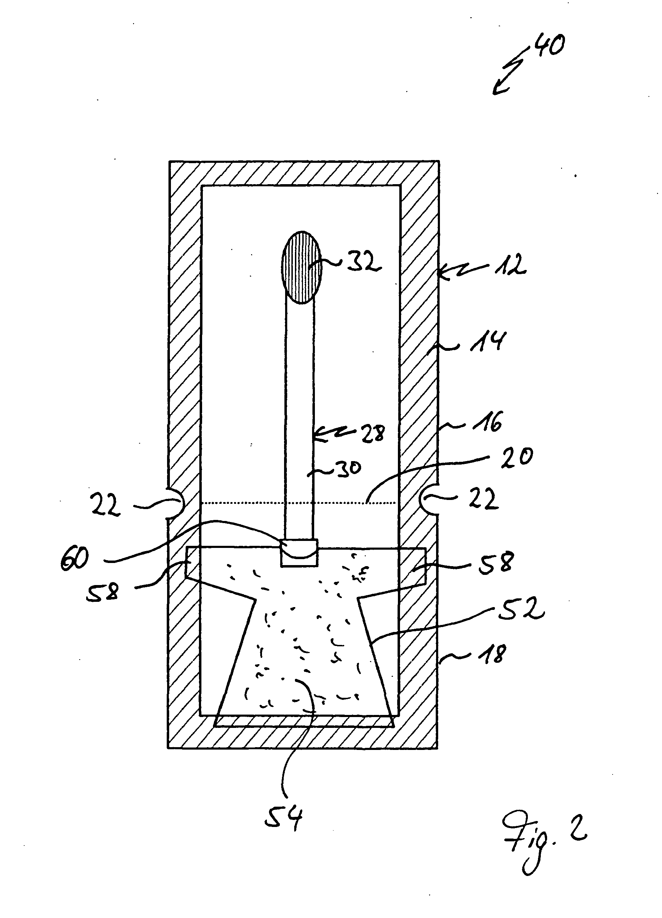

In Fig. 2 ist eine weitere Ausführungsform einer Vorrichtung 40 zum Auftragen eines flüssigen Stoffes auf eine Applikationsfläche dargestellt. Die Vorrichtung 40 entspricht im Wesentlichen derjenigen nach Fig. 1, unterscheidet sich von dieser aber dadurch, dass der Applikationsabschnitt 32 des Applikators 28 im Trockenbereich der Verpackung 12 liegt und der Griffabschnitt 30 mit seinem dem Applikationsabschnitt 32 abgewandten Endabschnitt 60 die Öffnung des Behälters bzw. Röhrchens 52 nach Art eines Stopfens verschließt. Die Öffnung kann in nicht dargestellter Weise mit einem Einführtrichter für das Pinselhaar des Applikators 28 aufweisen.

Zusätzlich hat der Behälter 52 zur Stabilisierung in der Verpackung 12 seitliche Fortsätze 58, die in den seitlichen Siegelnähten der Verpackung 12 verschweißt sind. Der Behälter ist damit in drei Bereichen, nämlich in seinem Fußbereich und im Bereich seiner seitlichen Fortsätze bzw. Flügel mit der Verpackung verbunden. Dies ist natürlich auch auf die Ausführungsform nach Fig. 1 übertragbar.

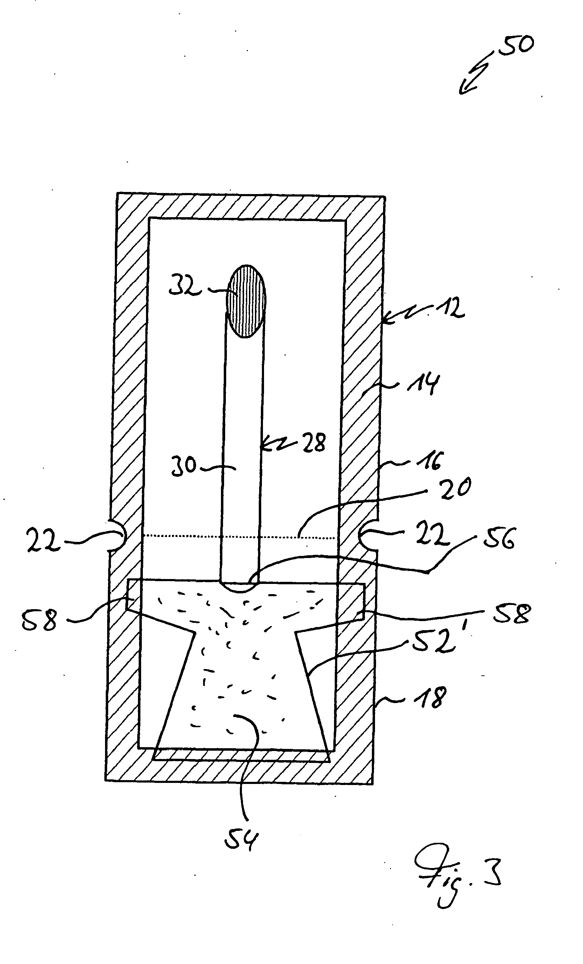

In Fig. 3 ist eine weitere Ausführungsform einer erfindungsgemäß ausgebildeten Vorrichtung 50 dargestellt, die im Wesentlichen derjenigen nach Fig. 2 entspricht, sich von dieser aber dadurch unterscheidet, dass der Applikator 28 einstückig mit dem ein Trennelement darstellenden Behälter 52' ausgebildet ist. Zur Aktivierung ist im Bereich der Öffnung des Behälters eine Sollbruchstelle 56 ausgebildet. Zur Aktivierung wird nach dem Aufreißen der beutelartigen Verpackung 12 der Applikator 28 an der Sollbruchstelle 56 von dem Behälter 52' abgetrennt, so dass dessen Öffnung freiliegt und der Applikationsabschnitt 32 des Applikators 28 zur Aufnahme des fließfähigen Stoffes in den Behälter 52' eingetaucht werden kann.

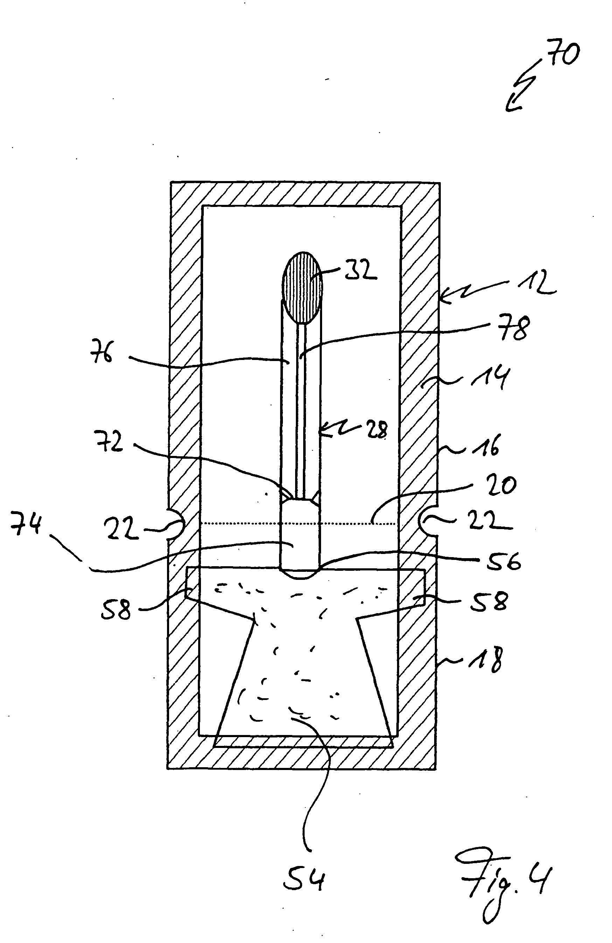

In Fig. 4 ist eine weitere Ausführungsform einer erfindungsgemäß ausgebildeten Vorrichtung 70 dargestellt, die im Wesentlichen derjenigen nach Fig. 3 entspricht, sich von dieser aber dadurch unterscheidet, dass im Bereich des Griffabschnitts 30 des Applikators 28 eine zweite Sollbruchstelle 72 vorgesehen ist, an der ein massiver Griffbereich 74 von einem Griffbereich 76 abgetrennt werden kann. Der Griffbereich 76 hat einen Axialkanal 78, der zu dem Applikationsbereich 32 bzw. Pinselhaar führt.

Bei der Anwendung der Vorrichtung 70 wird zunächst die Verpackung 12 entlang der Reißlinie 20 aufgerissen. Dann wird der Applikator im Bereich der ersten Sollbruchstelle 56 von dem Behälter 52' abgetrennt. Anschließend wird der massive Griffbereich 74 abgetrennt und der Applikator 28 mit dem Griffbereich 76 in den Behälter 52' über dessen Öffnung eingesteckt. Nun kann durch äußeren manuellen Druck auf den Behälter 52', der aus einem elastisch verformbaren Kunststoffmaterial besteht, der fließfähige Stoff aus dem Behälter 52' über den Axialkanal 78 des Griffbereichs 76 zu dem Applikationsabschnitt 32 gefördert und appliziert werden.

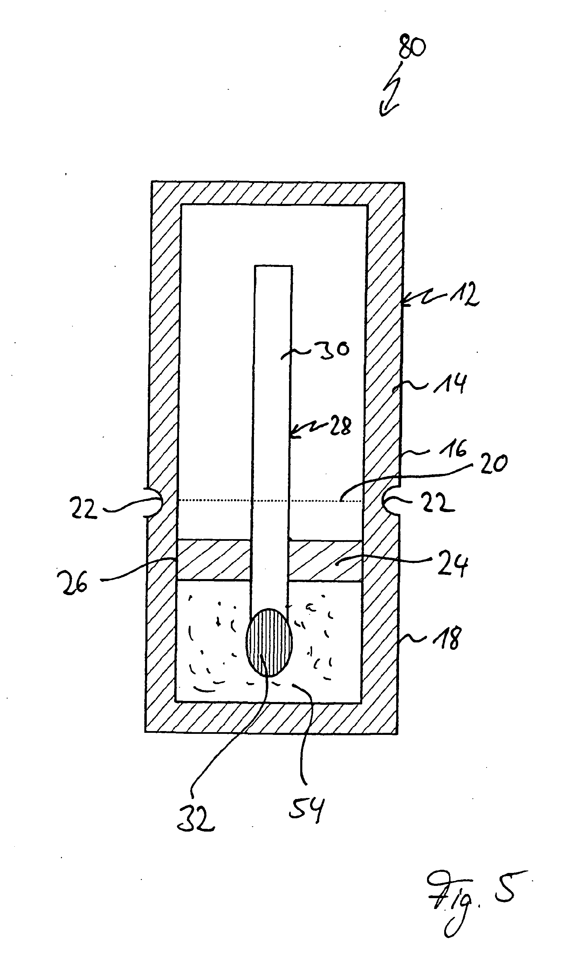

In Fig. 5 ist eine weitere Ausführungsform einer Vorrichtung 80 dargestellt, die zur Aufnahme und zum Applizieren eines fließfähigen Stoffes dient. Bei dieser Vorrichtung 80 ist zwischen dem Trockenbereich und dem Nassbereich des Weiteren ein ein Trennelement darstellender, scheibenförmiger Einsatz 24 angeordnet, das über eine in Querrichtung verlaufende Siegelnaht 26 mit der den Folienzuschnitten der beutelartigen Verpackung 12 verbunden ist.

Der im Grundriss linsenförmige Einsatz 24 ist von einer pinselartigen Applikationseinrichtung 28 durchgriffen, die einen in dem Trockenbereich bzw. Verschlussabschnitt angeordneten Griffabschnitt 30 und einen aus Pinselhaar gebildeten Applikationsabschnitt 32 umfasst. Der Applikationsabschnitt 32 taucht in den innerhalb des Aufnahmeabschnitts angeordneten Nassbereich 18, d. h. in den zu applizierenden Stoff ein. Der Einsatz 24 kann in nicht dargestellter Weise beidseits Laschen aufweisen, die in die seitlichen Randbereiche bzw. Siegelnähte des Verpackung eingeschweißt sind.

Im geschlossenen Zustand der Vorrichtung 10 steckt ein Bereich des Griffabschnitts 30 des Pinsels 28 formschlüssig im Presssitz in einer mittigen Ausnehmung 42 des Einsatzes 24, so dass keine Flüssigkeit aus dem Nassabschnitt 18 in den Trockenabschnitt 16 strömen kann. Der Durchmesser der Ausnehmung 42 korrespondiert mit dem Durchmesser des Griffabschnitts 30 der Applikationseinrichtung 28, so dass die Applikationseinrichtung 28 dichtend im Presssitz in dem Einsatz 24 gehalten ist.

### Bezugszeichen

- 10: Vorrichtung
- 12: Verpackung
- 14: Randbereich
- 16: Verschlussabschnitt
- 18: Aufnahmeabschnitt
- 20: Reißlinie
- 22: Kerben
- 24: Rohrstück
- 26: Siegelnaht
- 28: Applikator
- 30: Griffabschnitt
- 32: Applikationsabschnitt
- 40: Vorrichtung
- 42: Ausnehmung
- 50: Vorrichtung
- 52: Röhrchen
- 54: Aufnahmeraum
- 56: Sollbruchstelle
- 58: Fortsätze
- 60: Endabschnitt
- 70: Vorrichtung
- 72: Sollbruchstelle
- 74: Griffbereich
- 76: Griffbereich
- 78: Axialkanal
- 80: Vorrichtung

## Patentansprüche

1. Vorrichtung zum Auftragen eines flüssigen, pastenartigen oder anderen fließfähigen Stoffes, umfassend eine Verpackung (12) mit einem einen Nassbereich aufweisenden Aufnahmeabschnitt (18) zur Aufnahme des Stoffes und einem einen Trockenbereich begrenzenden Verschlussabschnitt (16), der über eine Reißlinie (20) von dem Aufnahmeabschnitt (18) getrennt ist, wobei der Nassbereich von dem Trockenbereich mittels eines Trennelements (52) getrennt ist, das eine durch ein Verschlusselement (28) verschlossene Öffnung aufweist, das nach einem Aufreißen der Verpackung (12) entlang der Reißlinie (20) von dem Trennelement (52) lösbar ist, **dadurch gekennzeichnet, dass** das Trennelement (52) ein einen Aufnahmeraum (54) begrenzender Behälter ist, der eine Primärverpackung für den fließfähigen Stoff bildet, und dass die Verpackung eine Sekundärverpackung für den fließfähigen Stoff bildet, wobei das Trennelement (52) in mindestens einem Bereich in eine Siegelnaht der Sekundärverpackung eingeschweißt ist.

2. Vorrichtung nach Anspruch 1, **dadurch gekennzeichnet, dass** das Verschlusselement ein Applikator (28) ist, der von der Verpackung (12) vollständig aufgenommen ist und mit einem Applikationsabschnitt (32) und einem Griffabschnitt (30) versehen ist, der in dem Trockenbereich der Verpackung (12) liegt, wobei die Öffnung des Trennelements (52) zum Eingriff oder zur Verbindung des eines Applikationsabschnitts (32) des Applikators (28) in den bzw. mit dem Nassbereich ausgelegt ist.

3. Vorrichtung nach Anspruch 1, **dadurch gekennzeichnet, dass** das Trennelement (52) von einem einseitig geschlossenen Rohrstück gebildet ist, das den Aufnahmeraum (54) begrenzt und im Bereich seines geschlossenen Endes mit der Verpackung verbunden ist.

4. Vorrichtung nach einem der Ansprüche 2 bis 4, **dadurch gekennzeichnet, dass** der Applikationsabschnitt (32) des Applikators (28) in den Nassbereich eintaucht und der Griffabschnitt (30) des Applikators (28) die Öffnung des Trennelements (52) dichtend durchgreift.

5. Vorrichtung nach einem der Ansprüche 2 bis 4, **dadurch gekennzeichnet, dass** der Applikationsabschnitt (32) des Applikators (28) in dem Trockenbereich liegt und der Griffabschnitt (30) des Applikators (28) mit seinem dem Applikationsabschnitt abgewandten Ende die Öffnung des Trennelements (52) verschließt.

6. Vorrichtung nach Anspruch 6, **dadurch gekennzeichnet, dass** der Griffabschnitt (30) einen Verschlussstopfen für die Öffnung des Trennelements (52) bildet.

7. Vorrichtung nach Anspruch 6, **dadurch gekennzeichnet, dass** der Griffabschnitt (30) einstückig mit dem Trennelement (52') hergestellt ist und im Bereich der Öffnung über mindestens eine Sollbruchstelle von dem Trennelement (52') getrennt ist, wobei ein Abtrennen des Applikators (28) von dem Trennelement (52) dessen Öffnung freigibt.

8. Vorrichtung nach einem der Ansprüche 1 bis 8, **dadurch gekennzeichnet, dass** die Verpackung (12) aus zwei in ihren Randbereichen umlaufend miteinander versiegelten Folienzuschnitten gebildet ist.

9. Vorrichtung nach Anspruch 9, **dadurch gekennzeichnet, dass** die Folienzuschnitte jeweils aus einer Verbundfolie gebildet sind.

10. Vorrichtung nach Anspruch 10, **dadurch gekennzeichnet, dass** die Verbundfolie ein Aluminiumlaminat ist.

11. Vorrichtung nach einem der Ansprüche 9 bis 11, **dadurch gekennzeichnet, dass** die Folienzuschnitte in Höhe des Trennelements eine quer zur Achse des Applikators (28) verlaufende Siegelnaht (26) aufweisen.

12. Vorrichtung nach einem der Ansprüche 9 bis 12, **dadurch gekennzeichnet, dass** die Reißlinie (20) durch mindestens eine Aufreißkerbe (22) im Bereich einer Siegelnaht der Folienzuschnitte festgelegt ist.

13. Vorrichtung nach Anspruch 2, **dadurch gekennzeichnet, dass** das Trennelement von einem mit der Verpackung (12) versiegelten, scheibenförmigen Kunststoffeinsatz gebildet ist, der von dem Applikator (28) durchgriffen ist.

14. Vorrichtung nach Anspruch 14, **dadurch gekennzeichnet, dass** der Kunststoffeinsatz einen linsenförmigen Grundriss aufweist.

15. Vorrichtung nach einem der Ansprüche 2 bis 15, **dadurch gekennzeichnet, dass** der Applikator (28) pinselartig ausgebildet ist und der Applikationsabschnitt (32) von Pinselhaar, einer Beflockung, einem Schaumstoff oder dergleichen gebildet ist.

16. Vorrichtung nach Anspruch 16, **dadurch gekennzeichnet, dass** das Pinselhaar an den Griffabschnitt angespritzt ist.

17. Vorrichtung nach einem der Ansprüche 2 bis 17, **dadurch gekennzeichnet, dass** der Griffabschnitt (30) einen Axialkanal (78) aufweist.

## Claims

1. Device for applying a liquid, pasty or other flowable substance, comprising a pack (12) with a receiving section (18) comprising a wet portion for receiving the substance and a closure section (16) defining a dry portion and being separated from the receiving section (18) by a tear line (20), said wet portion being separated from the dry portion by means of a separation element (52), comprising an orifice being closed off by a closure element (28) which is releasable from the separation element (52) after tearing open the pack (12) along the tear line (20),
**characterized in that**
the separation element (52) is a receptacle defining a receiving space (54), forming a primary pack for the flowable substance, and the pack forming a secondary pack for the flowable substance, wherein at least one portion of the separation element (52) is welded in a sealing seam of the secondary pack.

2. Device according to Claim 1,
**characterized in that**
the closure element is an applicator (28) being fully incorporated in the pack (12) and featuring an application section (32) and a finger grip section (30) located in the dry portion of the pack (12), wherein the orifice of the separation element (52) is designed to engage or connect the application section (32) of the applicator (28) in and with the wet portion respectively.

3. Device according to Claim 1,
**characterized in that**
the separation element (52) is composed of a tubular part being closed off at one end, defining the receiving space (54) and being connected to the pack proximal its closed end.

4. Device according to any of Claims 2 to 3,
**characterized in that**
the application section (32) of the applicator (28) dips into the wet portion and the finger grip section (30) of the applicator (28) sealingly passes through the orifice of the separation element (52).

5. Device according to any of Claims 2 to 3,
**characterized in that**
the application section (32) of the applicator (28) is located in the dry portion and the finger grip section (30) of the applicator (28) closes off the orifice of the separation element (52) by its end facing away from the application section.

6. Device according to Claim 5,
**characterized in that**
the finger grip section (30) forms a plug for the orifice of the separation element (52).

7. Device according to Claim 5,
**characterized in that**
the finger grip section (30) is produced in one piece with the separation element (52') and is separated from the separation element (52') in the area of the orifice by at least one predetermined breaking point so that parting the applicator (28) from the separation element (52) releases the orifice.

8. Device according to any of Claims 1 to 7,
**characterized in that**
the pack (12) is formed by two foil blanks being edge-sealed on all sides.

9. Device according to Claim 8,
**characterized in that**
the foil blanks are each made of a composite film.

10. Device according to Claim 9,
**characterized in that**
the composite film is an aluminum laminate.

11. Device according to any of Claims 8 to 10,
**characterized in that**
the foil blanks feature a sealing seam (26) oriented crosswise to a centerline of the applicator (28) and level with the separation element.

12. Device according to any of Claims 8 to 11,
**characterized in that**
the tear line (20) is defined by at least one tear nick (22) proximal a sealing seam of the foil blanks.

13. Device according to Claim 2,
**characterized in that**
the separation element is formed by a disc-shaped plastic insert being sealed with the pack (12) and being passed through by the applicator (28).

14. Device according to Claim 13,
**characterized in that**
the plastic insert features a lenticular configuration.

15. Device according to any of Claims 2 to 14,
**characterized in that**
the applicator (28) is configured brush-like and the application section (32) is formed by brush hair, a flock coating, a foam material or the like.

16. Device according to Claim 15,
**characterized in that**
the brush hair is moulded to the finger grip section.

17. Device according to any of Claims 2 to 16,
**characterized in that**
the finger grip section (30) comprises an axial passageway (78).

## Revendications

1. Dispositif pour l'application d'une substance liquide, pâteuse ou d'une autre substance coulante, comprenant un emballage (12) avec une section réceptrice (18) présentant une zone humide pour recevoir la substance et une section de fermeture (16) définissant une zone sèche et étant séparée de la section réceptrice (18) par une ligne de déchirement (20), ladite zone humide étant séparée de la zone sèche au moyen d'un élément de séparation (52) présentant un orifice qui est fermé par un élément de fermeture (28) qui peut être détaché de l'élément de séparation (52) après avoir déchiré l'emballage (12) le long de la ligne de déchirement (20),
**caractérisé en ce que**
l'élément de séparation (52) est un récipient définissant un espace de réception (54) formant un emballage primaire pour la substance coulante, et l'emballage formant un emballage secondaire pour la substance coulante, dans lequel l'élément de séparation (52) dans au moins une région étant soudé dans un joint de scellement de l'emballage secondaire.

2. Dispositif selon la revendication 1,
**caractérisé en ce que**
l'élément de fermeture est un applicateur (28) qui est complètement reçu par l'emballage (12) et qui est muni d'une section d'application (32) et d'une section de poignée par les doigts (30) située dans la zone sèche de l'emballage (12), dans lequel l'orifice de l'élément de séparation (52) est conçu de manière à ce que la section d'application (32) de l'applicateur (28) s'engrène dans ou est relié avec la zone humide respectivement.

3. Dispositif selon la revendication 1,
**caractérisé en ce que**
l'élément de séparation (52) est formé par une partie tubulaire étant fermée d'un côté et définissant l'espace de réception (54) et dans la région de son bout fermé étant relié avec l'emballage.

4. Dispositif selon l'une quelconque des revendications 2 à 3,
**caractérisé en ce que**
la section d'application (32) de l'applicateur (28) plonge dans la zone humide et la section de poignée par les doigts (30) de l'applicateur (28) passe à travers l'orifice de l'élément de séparation (52) de manière étanche.

5. Dispositif selon l'une quelconque des revendications 2 à 3,
**caractérisé en ce que**
la section d'application (32) de l'applicateur (28) est située dans la zone sèche, et la section de poignée par les doits (30) de l'applicateur (28) avec son extrémité étant détournée de la section d'application ferme l'orifice de l'élément de séparation (52).

6. Dispositif selon la revendication 5,
**caractérisé en ce que**
la section de poignée par les doigts (30) forme un bouchon pour l'orifice de l'élément de séparation (52).

7. Dispositif selon la revendication 5,
**caractérisé en ce que**
la section de poignée par les doigts (30) est fabriquée d'un seul tenant avec l'élément de séparation (52') et dans la région de l'orifice est séparée de l'élément de séparation (52') par au moins une point de rupture, dans lequel lorsque l'applicateur (28) est séparé de l'élément de séparation (52) l'orifice est libéré.

8. Dispositif selon l'une quelconque des revendications 1 à 7,
**caractérisé en ce que**
l'emballage (12) est formé de deux feuilles découpées dont les bords sont scellés de tous côtés.

9. Dispositif selon la revendication 8,
**caractérisé en ce que**
les feuilles découpées sont chacune fabriquée d'une feuille laminée.

10. Dispositif selon la revendication 9,
**caractérisé en ce que**
la feuille laminée est une feuille laminée en aluminium.

11. Dispositif selon l'une quelconque des revendications 8 à 10,
**caractérisé en ce que**
les feuilles découpées, au niveau de l'élément de séparation, présentent un joint de scellement (26) étant orienté transversalement par rapport à l'axe central de l'applicateur (28).

12. Dispositif selon l'une quelconque des revendications 8 à 11,
**caractérisé en ce que**
la ligne de déchirement (20) est définie par au moins une encoche de déchirement (22) à proximité du joint de scellement des feuilles découpées.

13. Dispositif selon la revendication 2,
**caractérisé en ce que**
l'élément de séparation est formé par un insert en plastique étant conçu sous la forme d'un disque scellé avec l'emballage (12) et étant pénétré par l'applicateur (28).

14. Dispositif selon la revendication 13,
**caractérisé en ce que**
l'insert en plastique présente une construction lenticulaire.

15. Dispositif selon l'une quelconque des revendications 2 à 14,
**caractérisé en ce que**
l'applicateur (28) est conçu sous la forme d'une brosse et la section d'application (32) est formée par des poils de brosse, un revêtement floqué, un matériau en mousse ou quelque chose de similaire.

16. Dispositif selon la revendication 15,
**caractérisé en ce que**
les poils de brosse sont moulés à la section de poignée par les doigts.

17. Dispositif selon l'une quelconque des revendications 2 à 16,
**caractérisé en ce que**
la section de poignée par les doigts (30) présente un passage axial (78).
